# EUROPEAN PATENT APPLICATION

(11) **EP 3 792 924 A1**
(43) Date of publication of application: **17.03.2021**
(21) Application number: 19197083.9
(22) Date of filing: 12.09.2019
(51) Int. Cl.: G16B 50/20, G16B 50/30, G06F 8/34

(54) **A SYSTEM AND METHOD FOR BIOINFORMATICS DATA ANALYSIS BY PROCESSING COMPUTATIONAL DATA ANALYSIS WORKFLOWS**

(71) Applicant: Bluebee Holding B.V., 2289 DC Rijswijk (NL)
(72) Inventor: Sima, Vlad-Mihai, 2289 DC Rijswijk (NL)
(74) Representative: Robinson, David Edward Ashdown

(57) **Abstract**

A system and method for bioinformatics data analysis by processing computational data analysis workflows. A computational workflow formed by a graph is received. The graph has a graph structure with nodes and edges, and the edges in the graph indicate a flow of data between linked nodes. Further, input data is obtained and processing steps are performed on the input data by execution of nodes of the graph structure, wherein processed result data from the processing steps is stored. A node configuration update of a selected node is performed based upon a received node configuration adjustment by the user based upon a received graph structure adjustment by the user, wherein the node configuration update and/or graph structure update are carried out while performing the processing steps during processing of the computational data analysis workflow. Processing of the computational data analysis workflow is continued based on updated node configuration of the selected node and/or updated graph structure using already processed result data being available.

## Description

### FIELD OF THE INVENTION

The invention relates to a bioinformatics data analysis system for processing computational data analysis workflows. The invention further relates to a computer implemented method for bioinformatics data analysis by processing computational data analysis workflows. Furthermore, the invention relates to a computer program product for bioinformatics data analysis.

### BACKGROUND TO THE INVENTION

Bioinformatics analysis, such as e.g. genomic analysis, is a widely used technology. Analytical data analysis tools may be arranged into a graph (also known as pipeline). For this purpose, a computer program product may be used. These graphs may also be used as standalone tools in further building of other graphs. The graph or pipeline may represent a complex analytical algorithm including any combination of existing tools which are typically represented by nodes. Some nodes can be interconnected by means of edges defining flow of data between said nodes. The graph can be assembled, represented and executed within a graph/pipeline editor. Such editor may appear on a graphical user interface allowing for intuitive assembly of the nodes (cf. tools), edges and further specification of the nodes/edges into the data analysis pipeline by the user.

Typically a bioinformatics analysis is performed in a workflow execution platform. This may for instance be a cloud-based environment providing a way to retrieve stored data, analyze the data and interpret the bioinformatics derived data. Result data may be stored for further processing and/or interpretation. The workflow may include one or more graphs defining the processing for the datasets (e.g. genomics data, transcriptomics data, etc.). Each node of the graph may include one or more processing steps to be carried out on data retrieved by the node. This retrieved data may already have been processed to some extent by a previous node. However, it is also possible that the retrieved data comes directly or indirectly from a data storage (e.g. database).

The platform may include a graphical user interface which is configured to allow a user to create projects, run tasks, upload data, adapt existing projects, create or adapt program modules or nodes of the graph, etc. Additionally or alternatively, the platform may include an application programming interface which is configured to allow for (processing) procedures to be carried out programmatically with additional logic. Further, a command line interface can be provided configured to allow for interactive control of the graph or pipeline. Various solution are possible. For example, the system can be adapted to connect to a knowledge structure for obtaining input data. The knowledge structure may for instance include one or more databases. In some cases, the knowledge structure is cloud-based.

Typically, bioinformatics data analysis involves processing of extremely large data sets. This may require a large amount of processing time for obtaining result data from the retrieved input data. However, as a result, the development of a pipeline can also become a cumbersome process. It may be difficult to efficiently adapt the pipeline. As a result, adapting the graph may be very labor intensive.

There is a desire to optimize processing of bioinformatics data by means of graphs. Therefore, there is a need to obtain a more efficient system for analyzing large data sets. It is also desired to allow for easy retrieval and analysis of large amounts of data, while simplifying the process.

### SUMMARY OF THE INVENTION

It is an object of the invention to provide for a method and a system that obviates at least one of the above mentioned drawbacks.

Additionally or alternatively, it is an object of the invention to improve bioinformatics data analysis.

Additionally or alternatively, it is an object of the invention to provide a more efficient processing of bioinformatics data, especially when larger data sets are involved.

Thereto, the invention provides for a bioinformatics data analysis system for processing computational data analysis workflows, the data analysis system comprising a computing unit including a processor coupled to a memory operable to cause the system to: receive a computational workflow formed by a graph, wherein the graph has a graph structure with nodes and edges, wherein the edges in the graph structure indicate a flow of data between linked nodes, and wherein the nodes have a node configuration including at least a set of parameters and/or operational tasks, wherein the node configuration of the nodes and/or the graph structure are adjustable by a user; obtain input data and perform processing steps on the input data by execution of nodes of the graph structure, wherein processed result data from the processing steps is stored; perform a node configuration update of a selected node based upon a received node configuration adjustment by the user, and/or perform a graph structure update based upon a received graph structure adjustment by the user, wherein the node configuration update and/or graph structure update are carried out while performing the processing steps during processing of the computational data analysis workflow; and continue processing of the computational data analysis workflow based on updated node configuration of the selected node and/or updated graph structure using at least a portion of already processed result data being available.

The processing steps resulting from the execution of a node of the graph can depend on the configuration (e.g. parameters, operational tasks or programming code) and/or the graph structure of the node. The configuration of the nodes and/or the graph structure can be changed or adapted during execution of the graph process, or even during execution of the particular node which is edited. Already processed data stored as a result of processing prior to performing the change/update can be used in the further processing using the updated configuration and/or graph structure. Advantageously, a better control can be obtained over the operation or functionality of the graph. The updates can be performed by a user. However, automatic adjustments (e.g. programmed) are also possible.

The graph can be more efficiently adapted during processing, i.e. while the data analysis is still running. While the one or more nodes of the graph are being executed, the configuration of said one or more nodes can be modified, avoiding that the (complete) process has to be restarted. In this way, the graph can be more efficiently transformed or edited. Hence, the functionality and parameters of the nodes can also be better fine-tuned. Often, the analysis of the large data sets requires changing of the graph configuration, settings, functionality and/or parameters of the graph or its components while the execution process has already started. The invention enables using the already processed result data when the process is continued.

The invention provides an effective method for processing bioinformatics workflows including an analysis graph/pipeline. The nodes of the graph can be independently operable with respect to each other. Input data can be provided to a chain of sub-processes or modules represented by the nodes of the graph, wherein one or more outputs are obtained at the node providing characterization of the input data provided to the node. The nodes are re-(re)configurable or customizable during the running process providing a dynamic control of the workflow execution. Advantageously, already processed data can be used being available at a time of continued execution of the selected node with updated node configuration. Hence, the available result data previous to reconfiguration of the selected node does not need to be discarded and may be used for further analysis by the graph. The execution process can be continued until the computational workflow ends, or has been actively halted or restarted.

A complex data analysis (cf. workflow) can be performed on the basis of a graph (cf. network) of nodes (cf. components of network). Each node may encapsulate a complete programming environment (cf. scientific notebook) defining a plurality of operational tasks when the node is executed. The inputs and outputs of the node can be defined both programmatically and visually (e.g. using a graph editor or another graphical user interface). Optionally, inputs and outputs of the node can be defined dynamically, for instance after the node has been placed in the graph. The input data provided to the node can be made available automatically for each node. The components can be run (and edited) interactively and/or in the background. In case of background execution, each component can have a graphical interface to reflect the status of the execution. Optionally, in case of background execution, each component can have an input interface which allows for accepting the current result data (e.g. for machine learning algorithms that improve over time) or fail the node by discarding at least a portion of the result data already available.

Optionally, the system is configured to listen for node configuration adjustments. For instance, such adjustment may be detected when a certain event is triggered. After receiving the node configuration adjustment, the corresponding change can be implemented in the computational workflow process.

The processing of the computational data analysis workflow and the execution of the nodes can be continued based on intermediate results (cf. result data) already determined, such that tuning of node configurations (e.g. parameters, code, etc.) can be more efficiently improved in order to reduce the total time required for performing the data analysis.

The graph structure can be adjusted or modified in different ways. For instance, a node can be added, replaced, moved, shifted, copied, repositioned, cloned, re-arranged, etc. In some examples the relative arrangement of the nodes and edges are changed when adjusting the graph structure.

Optionally, the system is configured to enable a user to examine a status of each node while performing the processing steps during the processing of the computational data analysis workflow.

The node status can provide additional information of the current running process of the node being executed. The status of the node can provide an interface to interact with the executing node. Advantageously, the state of each node (cf. sub-process) in the graph can be looked into in real-time during the workflow processing.

If one of the nodes being executed is for example running too slow or inefficiently, the configuration of the node can be adjusted in order to solve this issue. Therefore, once the processing of the graph has been started, it is not required to restart it completely when the graph has been changed and/or when node configurations have been updated.

The status of the nodes can be continuously monitored by a user during the data processing analysis. If a condition in the configuration of a node is too strict, the configuration, e.g. parameter or program code, can be adequately changed by the user in order to allow the process to continue in an improved way.

Optionally, execution of the node can be aborted by the user. The already processed result data being available may then be subsequently used when continuing processing in the graph. Advantageously, more control over the processing of the computational data analysis workflow can be obtained.

Optionally, the system further provides for a graph editor, wherein the graph editor provides a functionality for editing, running and viewing a status of the selected node.

Optionally, the system further provides for a programming editor when the node is edited, wherein the programming editor enables changing of the program code of the node. The program code can relate to or represent the operational tasks of the node configuration.

Optionally, the system is configured to enable a user to skip further execution of an active node by discontinuing execution of the active node and continuing execution of a subsequent node in the graph using the already processed result data being available.

When the graph is being executed (i.e. 'running'), different nodes can be performing processing tasks. While the graph is running, it is possible execute a node independently. The system can enable monitoring of the state of the node and occasionally skip further execution of the node, so that for example execution of a subsequent node can be initiated. This is especially advantageous for longer processes. Hence, a condition that one node has to finish before a subsequent node is executed can be overruled in this way (cf. override). It is possible to interact with the previous condition set for further execution of the subsequent node. This condition may be part of the node configuration, for instance implemented as a parameter or implemented as a code in the operational tasks of the node.

Advantageously, according to the invention, it is not required to run a graph from start to end. For instance when a processing of a node being executed has already finished for 90%, it can be considered good enough and further execution of the node can be skipped. However, the already processed result data being available can eventually, at least in part, be used by one or more of the subsequent nodes in the graph.

For example, one of the nodes may have taken already an extensive amount of time of processing without finishing. This can be resolved by adjusting one of the parameters, or adjusting some operational tasks (e.g. adding lines of code in the code of the node). The node configuration can be changed and the graph can continue processing with the updated node configuration.

Optionally, upon updating the node configuration of a selected node, the system dynamically adjusts dedicated computational resources for performing the processing steps during the processing of the computational data analysis workflow.

A resource management can enable dynamic adjustment based on execution and/or user requirements. In some examples, the system includes a backend resource manager.

If a selected node is edited, for instance by changing its node configuration (e.g. its operational tasks by editing the code/script linked to the node), more or less resources may be required. For example, a bigger matrix may be defined, so that more memory may be required for performing operational tasks of the selected node. The system may for instance be configured to determine how much memory is required for the change of node configuration, and dynamically adjust the resources upon change of the node. If needed, more memory, processing power, etc. may be reserved for the node.

The resource allocation can be dynamically adjusted even while the processing steps are performed during processing of the computational data analysis workflow. The system may be configured to perform a dynamic resource allocation upon updating of the node configuration of the selected node. In some examples, the system may provide for a check whether sufficient resources are available prior to updating the node configuration of the selected node. In this way, it can be prevented that the processing of the computational data analysis workflow freezes due to insufficient resources.

Optionally, the system is further configured to determine whether the selected node in the graph has already been processed, wherein at least in case execution of the selected node in the graph is still ongoing or pending, processing of the computational data analysis workflow is continued based on the updated node configuration of the selected node.

The configuration can thus be adjusted 'on-the-fly' during processing even while the execution of the particular node is carried out. In this way the execution process can be adapted dynamically, providing important advantages for bioinformatics data analysis in which there is often a need for fine tuning or tweaking configurations, settings, program code etc. of the graph.

Optionally, in case the selected node has already been processed, the updated node configuration is used when the selected node is subsequently executed. The updated configuration may for example be used in a subsequent run of the graph.

Optionally, the system is configured to return processing of the computational data analysis workflow to execution of the selected node in case the selected node has already been processed.

The system may determine whether the execution of selected first node in the computational data analysis workflow has already been finished. When the node has already been executed and the process is completed, the processing may return to the relevant node and discard later result data (cf. rewind execution).

Optionally, the system is further operable by the user to change at least one of the nodes or edges of the graph while performing the processing steps during processing of the computational data analysis workflow, wherein processing of the computational data analysis workflow is continued based on the adjusted at least one of the nodes or edges of the graph while using at least a portion of already processed result data being available.

While processing steps are performed during processing of the computational analysis workflow, nodes can be added, removed , relocated, edited, etc.

Optionally, the bioinformatics data analysis system is configured to execute a sequence analysis pipeline.

Optionally, the graph includes at least one node relating to machine learning (ML).

A ML 'training node' can be configured to take a set of samples as input and output a trained neural network. A machine learning 'predictor node' can be configured to take a trained neural network node and a sample as input and perform a prediction as the output. Adaptively editing node configurations is especially advantageous in complex data analysis workflows, for instance including a plurality of machine learning (e.g. neural network) nodes in the graph.

Advantageously, ML nodes in bioinformatics graphs can work in a controllable way, allowing adjustments and/or tweaking even after some execution.

The changing of processing configurations (e.g. parameters) may be especially required for performing ML-type of work in the bioinformatics analysis. The ML parameters often require further fine tuning once the processing has started. In this way, relevant features can be extracted from biological data more efficiently, not requiring to restart the processing of the whole graph.

Optionally, the at least one node relating to machine learning is at least one of: a training node configured to take an input and output a trained model, such as a neural network; or a predictor node configured to take a trained model, such as a neural network, as input for obtaining a prediction as output. It will be appreciated that the machine learning nodes can be other types as well, such as for instance feature selection nodes, validation nodes, etc., or a combination of ML types. Many possibilities are envisaged.

For example, parameters of ML training of a neural network may be adapted without needing to restart the complete process. Already processed result data that is available can be used.

In ML training, a prediction of the neural network can be compared with the actual result. The difference allows to perform a step in the right direction for obtaining better predictions (different algorithms can be employed for this purpose). The size of the step taken in the right direction can for instance be one parameter that can be changed for improving the machine learning process. The configuration and/or settings of the graph (e.g. nodes) can be dynamically changed. Subsequently, the execution of the graph can be continued with the changed dynamic configuration and/or settings.

Optionally, the system may perform an issue (e.g. error, infinite loop, etc.) check for determining whether there are incompatibilities rising from the change of code.

Optionally, the system is further configured to perform a compatibility check when operational tasks of the selected node are adjusted, wherein the compatibility check includes comparing at least data inputs provided through edges to the selected node in a reconfigured state to that of a previous state prior to the reconfiguration.

Optionally, it is determined whether adjusted operational tasks define required inputs and/or outputs to linked nodes, as defined in the previous version of operational tasks. In some examples, incompatible adjustments to the node or its configuration are dismissed automatically. A warning may be prompted to the user in such a case.

The system may further provide for a input-output manager configured to automatically select possible inputs/outputs when defined inputs and/or outputs in the updated operational tasks are not fully specified. In this way, a better compatibility may be performed. The edges connected to inputs and outputs can be dynamically generated upon a relevant change of the node configuration.

The computational data analysis workflow can define the computational relationship between nodes and edges for processing data. Depending on the configuration of the graph, nodes can be executed consecutively, in parallel or combination of both.

Optionally, the system checks whether the interfaces have remained the same after change of the code/execution. An improved compatibility can be obtained in this way, and the risk of further issues or errors can be reduced.

Optionally, a sanity check is performed in order to see if the code compiles. Some code changes may not allow further continuation of execution. This can be prevented using the sanity check and avoid fatal runtime errors.

Optionally, the graph is executed in an asynchronous process.

The graph may be an asynchronous process with different branches of edges with nodes and in certain situations one or more of the nodes can have problems for proceeding further. Multiple nodes may for instance be processed in parallel. While node is running (e.g. progressing slow towards its goal) the node configuration can be tweaked or fine tuned, allowing improved processing. The previous processed data being available can be at least in part used when continuing the processing. Hence, stopping and completely restarting the workflow analysis with updated configuration(s) is not required. The graph can be adapted in real-time during processing.

Optionally, the system provides for an interface in which the code of the nodes can be edited by the user. The interface may provide a representation of the nodes and edges of the graph. Each node may have operational tasks in the form of code associated therewith. The code can be edited even while performing the processing steps during the execution of the node. When changing of the code has finished, the node can be executed using the updated code.

Optionally, the workflow is communicatively coupled to one or more databases using a data interface. In some examples, the database is a medical record database, where the medical record database contains medical information to be processed by the system, and where the medical information comprises textual data. Other types of data or databases are also envisaged.

According to an aspect, the invention provides for a computer implemented method for bioinformatics data analysis by processing computational data analysis workflows, the method comprising the steps of: receive a computational workflow formed by a graph, wherein the graph has a graph structure with nodes and edges, wherein the edges in the graph indicate a flow of data between linked nodes, and wherein the nodes have a node configuration including at least a set of parameters and/or operational tasks, wherein the node configuration of the nodes and/or the graph structure are adjustable by a user; obtain input data and perform processing steps on the input data by execution of nodes of the graph, wherein processed result data from the processing steps is stored; perform a node configuration update of a selected node based upon a received node configuration adjustment by the user, and/or perform a graph structure update based upon a received graph structure adjustment by the user, wherein the node configuration update and/or graph structure update are carried out while performing the processing steps during processing of the computational data analysis workflow; and continue processing of the computational data analysis workflow based on updated node configuration of the selected node and/or updated graph structure using at least a portion of already processed result data being available.

Although the nodes of the graph can be pre-configured and/or preprogrammed upon initiation of the processing of the computational data analysis workflow, the configuration and/or programming of the nodes can be changed during runtime in which the processing steps are performed according to the computational data analysis workflow (cf. during processing). The process can be continued using already processed result data available. A more fluid control can be obtained over the processing of the computational data analysis workflows.

Additionally or alternatively, the graph structure can be changed during runtime (cf. while performing the processing steps).

According to an aspect, the invention provides for a computer program product configured for performing, when run on a controller, the steps of the method according to the invention.

The node configuration (e.g. parameters, operational tasks, program code, settings, etc.) can be changed promptly while processing the computational data analysis workflow. In some examples, the active node may check certain parameters at regular time intervals for updating its execution.

According to an aspect, the invention provides for a software system for performing data analysis using a graph. The graph may have a plurality of nodes and edges connecting nodes, wherein each node is represented by a scientific notebook defining properties and/or characteristics of the node. The scientific notebook may include node configurations with programmed operational tasks and parameters.

Each node can have programming code as part of its configuration, wherein the code can be edited and executed on a processing unit. During execution of the process defined by the nodes of the graph (i.e. execution of the analysis), the scientific notebook or configurations of the nodes can be adapted, even if the node is already being executed. Hence, the nodes can be dynamically adjusted on-the-fly or in real-time, and already processed data can be used further even if the node has been updated with other configurations or settings.

According to an aspect, the invention relates to a system for performing computations on genomic data comprising a processor and a non-transitory computer-readable storage medium storing instructions that, when executed by the processor, cause the system to perform the method according to the invention.

Optionally, the system is configured to perform computations for analyzing a genomic data set to determine whether the genomic data set exhibits a particular variant indicative of a carrier status.

According to an aspect, the invention provides for a genomics analysis platform for executing a sequence analysis graph (cf. pipeline). According to an aspect the invention relates to a method for processing bioinformatics workflows.

According to an aspect, the invention provides for a system for processing genetic sequence data from a genomic sequencing device, the system comprising: an input for receiving the genetic sequence data from a sequencing of a sample from a human subject by the genomic sequencing device, and a data analysis system according to the invention.

Advantageously, a dynamic adjustment of the node execution is enabled, wherein node configuration changes can be performed even if the node is being executed. A more efficient bioinformatics workflow can be obtained in this way.

It will be appreciated that the invention is not limited to bioinformatics analysis, but can be used for other fields also. For instance, the workflow or graph may relate to general data analysis, image processing, sound processing, artificial machine learning, data composition, data filtering, genomics matching, pattern recognition, etc. Any type of data processing may be performed employing the system and method according to the invention. Many kinds of architectures can be employed. The invention can provide significant advantages in case the analysis is exploratory requiring a lot of fine tuning of parameters.

An application programming interface (API) may be provided allowing for the user to design and configure e.g. complex, multi-stage, or large batch analyses without using the general user interface (GUI). In some examples, any action that can be taken using the GUI can be executed using the API, but the API allows for additional user-generated logic. Further, in some examples, an API client may be provided allowing the user to interact with the API. Additionally or alternatively, the user can interact with the API by means of a programming language, such as for example Python. Additionally or alternatively, the user can interact with the API via the terminal of their computer by using a command line program. It will be appreciated that API commands can be chained together to form an API script. In this way, the automation and integration of various service features can be enabled. The API may offer various advantages over the GUI via programmatic access and automation, allowing for the creation of complex processes and workflows that would be not be practicable or cost efficient to implement as a GUI feature.

Though the API has many advantages over other modes of interaction, it lacks several benefits conferred using the GUI. For example, The API may not be accessible to users having insufficient programming expertise. In some cases, the API includes features not present in the GUI, those interacting with the service only with its GUI are unable to take advantage of the advanced features. In some advantageous examples, the user can use a combination of a GUI and a programming language for interacting with the graph/pipeline design of the data analysis workflow.

Although the embodiments of the invention may comprise computer apparatus and processes performed in a computer apparatus, the invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program.

It will be appreciated that the data analysis according to the invention also includes artificial intelligence (AI) and machine learning. Other data processing steps or activities may also be understood as data analysis.

It will be appreciated that the operational tasks may represent a algorithm description in a programming language. In some examples, the operational tasks are defined as program code or a script. The operational tasks may for instance represent a computer program. Hence, the configuration adjustment also enables modifying the program represented by the node.

It will be appreciated that any of the aspects, features and options described in view of the system apply equally to the computer-implemented method and the described computer program product. It will also be clear that any one or more of the above aspects, features and options can be combined.

The invention thus conceived is susceptible of numerous modifications and variations, all of which are within the scope of the inventive concept.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a graph;
Fig. 2 shows a schematic diagram of an embodiment of a graph;
Fig. 3 shows a schematic diagram of an embodiment of a graph;
Fig. 4 shows a schematic diagram of an embodiment of a node configuration; and
Fig. 5 shows a schematic diagram of a method.

### DETAILED DESCRIPTION

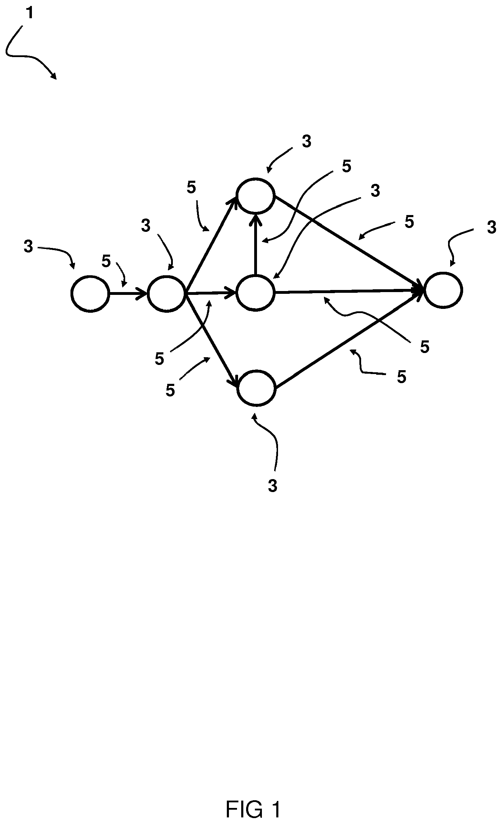

Fig. 1 shows a schematic diagram of an embodiment of a graph 1 in a bioinformatics data analysis system/method for processing computational data analysis workflows. A computational workflow can be formed by the graph 1. The graph 1 has a graph structure with nodes 3 and edges 5, wherein the edges 5 in the graph 1 indicate a flow of data between linked nodes. The nodes 3 have a node configuration including at least a set of parameters and/or operational tasks. The node configuration of the nodes and/or the graph structure are adjustable by a user. The nodes 3 can be linked to each other in various ways. Input data can be obtained, and processing steps can be performed on the input data by execution of nodes 3 of the graph structure. The processed result data from the processing steps can be stored. A node configuration update of a selected node and/or a graph structure update can be performed based upon a received node configuration adjustment by the user and/or a received graph structure adjustment by the user, respectively. The node configuration update and/or graph structure update can be carried out while performing the processing steps during processing of the computational data analysis workflow. Processing of the computational data analysis workflow can then be continued based on updated node configuration of the selected node and/or based on updated graph structure using at least a portion of already processed result data being available.

A node execution of a node 3 in a graph 1 can be changed by adjusting its configuration. The configuration can include one or more node parameters, or operational tasks for instance represented in programming code. The node configuration can be customized or adapted while one node is running (cf. during execution based on previous node configuration), wherein further processing can be continued with the updated configuration, while using already available result data.

Different types of parameters can be changed. For example, a threshold parameter of the node 3 can be changed while the node 3 is being executed, e.g. the threshold relating to a condition for proceeding further to a next step (e.g. subsequent node) in the graph 1.

For example, if during processing of the computational data analysis workflow, a small code change is desired, it is no longer needed to perform a labor-intensive process of finding the relevant part of the code, adapt the program code, package it with the updated code, and rerun the complete computational data analysis workflow from the start. Advantageously, the already processed result data being available can be used in an efficient way.

The invention provides significant advantages in bioinformatics analysis for which data sets are relatively large. For example, one sample may be 20 GB, so that having 5000 samples may correspond to a data set of 100 TB. The analysis may involve for example selecting some features of interest in the available input data. If too much features are selected in the first 100 samples, it is still possible to retain the work done, but a parameter can be adjusted (e.g. reduce a certain threshold) in order to be able to select more relevant features in the remaining samples to be processed. In this way, previously done processing can be saved and less resources and/or processing power may be needed to obtain an end result when processing of the graph/pipeline 1 has finished. Advantageously, the processing efficiency can be considerably increased.

Additionally or alternatively, in machine learning (ML), often parameters may need to be changed. For instance in training a neural network, some parameters in the neural network can be tweaked in order to obtain a better training. The invention provides for an effective way of dynamically changing node parameters in a graph 1, while retaining and using already processed result data being available.

Additionally or alternatively, the development of having a working graph 1 (with tuned parameters and node configurations) can be considerably improved. The process can be made significantly more efficient, as configurations and settings can be adjusted directly while continuing further processing using previous available result data. A better interaction with the system can be obtained.

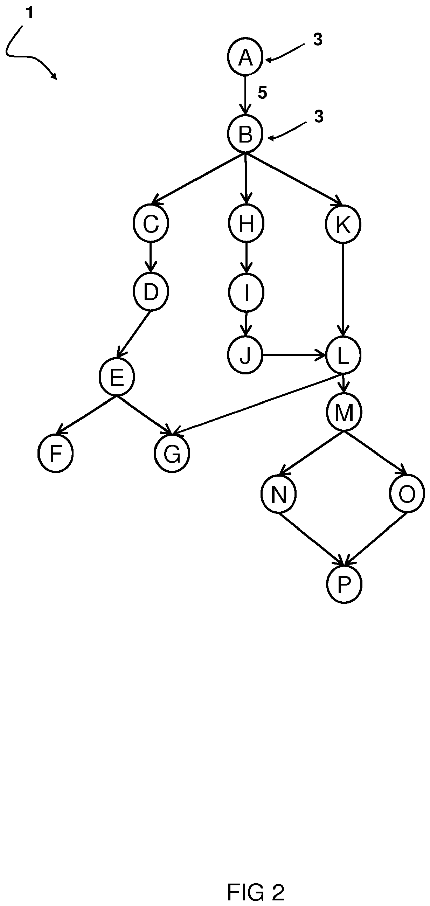

Fig. 2 shows a schematic diagram of an embodiment of a graph 1 with a plurality of nodes 3 and edges 5 connecting various nodes 3. The plurality of nodes are also labeled with letters A to P in the figure. As can be seen, some nodes can have multiple inputs and/or outputs. For example, node B has three outputs via which data is provided to nodes C, H and K. Node E provides data to nodes F and G. Furthermore, for example node M provides data to nodes N and O, while node P receives data from both nodes N and O. For example node L receives data from nodes J and K and outputs data to nodes M and G. It will be appreciated that various arrangements are possible.

The graph 1 may represent a chain of programmable sub-processes or modules represented by the nodes 3. The graph 1 may include a plurality of nodes connected to each other by means of edges 5. The nodes 3 may receive input data through edges connected thereto. A node 3 may produce an output based on input data, for example providing a characterization of the input data. The nodes (cf. sub-processes or modules) of the graph can be adjustable during the workflow processing. Advantageously, the nodes of the graph 1 can be re-configurable or customizable during processing. A selective re-adjustment of the configuration or arrangement of a node in the graph even during the workflow execution can provide various advantages as the system can provide for a dynamic control of the workflow execution.

When the graph 1 is executed, some nodes can be executed concurrently. When one node has difficulties executing, the execution at that node experiencing the difficulty can be intervened and the node configuration can be adjusted in order to allow continuation of execution at that particular node. It is also possible to skip further processing of the node. The intervention can advantageously be performed without needing to rerun the processing again from start, as already processed result data being available can be used.

The already processed stored data can be made available at the time of continued execution of the reconfigured node with updated node configuration. A dynamic configuration of the nodes 3 of the graph 1 can be provided.

The nodes 3 in the graph 1 can have different functionalities. The processing actions and functionalities of the node 3 can be changed by changing the node configuration. The nodes 3 may for instance perform pre-processing steps, relate to sequence matching, perform image processing, relate to machine learning, etc. Various data processing algorithm coupled to the nodes are envisaged.

In some examples, the graph structure is modified, even while the processing steps during processing of the computational data analysis workflow are being performed. The execution can continue with the updated graph structure, while using at least a portion of already processed result data being available.

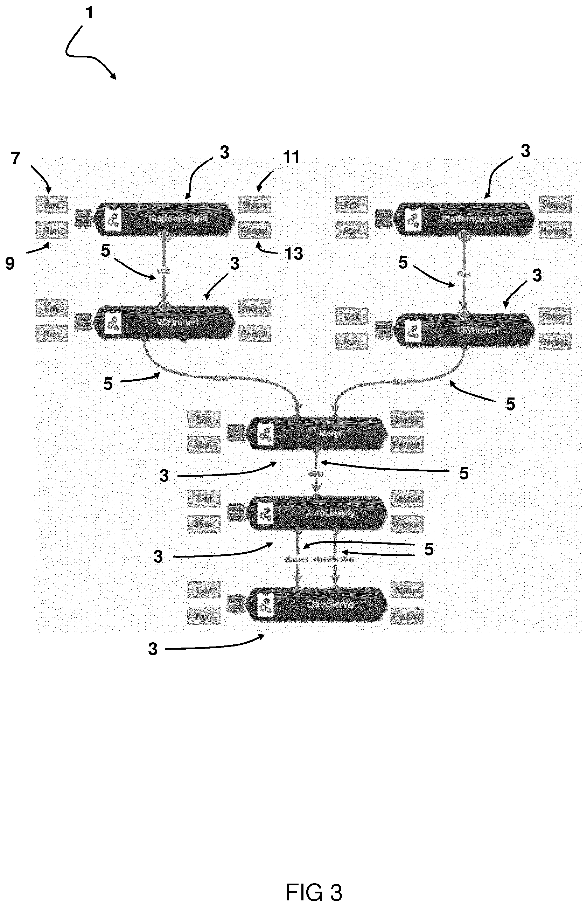

Fig. 3 shows a schematic diagram of an embodiment of a graph 1. The graph 1 has a plurality of nodes 3 connected to other nodes 3 by means of edges 5 providing a flow of data. The graph 1 is represented in a graphical user interface enabling the user to interact with i.a. the arrangement of the nodes and edges and the node configurations. The graphical user interface provides for an edit 7, run 9, status 11, and persist 13 button for each node 3. It will be appreciated that other buttons or functionalities may also be implemented. Advantageously, the user can easily check a node status and adjust the execution of the nodes while processing is performed.

The status check of a node 3 can be performed while the processing steps are performed during processing of the computational data analysis workflow. Even during execution of this particular node 3 the status of a particular node 3 can be checked. The status check can be used for determining a need of changing the execution of the particular node 3 being executed. For example, when a minimum accuracy of 95% is set as a condition for continuing with a subsequent node, and if an actual accuracy remains below 95% for a considerable amount of time, then a lot of processing time and/or resources may be wasted. By means of the status check, the user can better intervene in the execution process if needed. For example, the system enables a user to determine the status of the node 3, override the previous settings or configurations of the node 3, and continue the processing steps using already processed result data being available. It is possible to better understand the actual situation while the nodes are being executed. In this way, the user can better focus on a part of the graph that has to be modified in order to efficiently continue processing of the computational data analysis workflow if needed. It will be appreciated that the user can interact with the graph 1 in different ways.

The operational tasks may be a programming code of the node 3. Different programming languages or scripts can be used (e.g. Python, R, JavaScript, etc.). Also custom-made programming languages or scripts may be employed. In some examples the programming language defining the operational tasks of the node configuration is an interpreter language. However, other types of programming languages may also be employed. The program/code (cf. operational tasks) of the node can be changed even if the node is already being executed. For example, a portion of the graph 1 may already have been finished executing, and when an issue (e.g. error, infinite loop, non-converging result, too slow progress, etc.) occurs at a particular node, the operational tasks and/or parameters of the particular node can be changed and the processing can be efficiently continued using already processed result data being available from earlier processing of the graph 1.

It will be appreciated that the edges 5 in the graph 1 may represent all kind of data transfer, for instance, variables, strings, integers, files, etc.

In some examples, the operational tasks or programming code of the node 3 can also control the number of inputs and outputs. Changing the configuration of the node 3 can also be represented visually in the graph 1 by the graphical user interface. The functionality of the node 3 is not fixed, and also inputs-outputs can be dynamically changed, even while performing the processing steps during processing of the computational data analysis workflow. For example, different inputs and/or outputs can be added/removed to an existing node, and this can be implemented in the programming code linked to the node(s). The changed graph 1 can be run using already processed result data being available.

Additionally or alternatively, the graph structure may be changed/modified, for instance triggered by a user interaction. As a result, a graph structure update may be performed. The processing can then continue based on the graph structure using at least a portion of already processed result data being available.

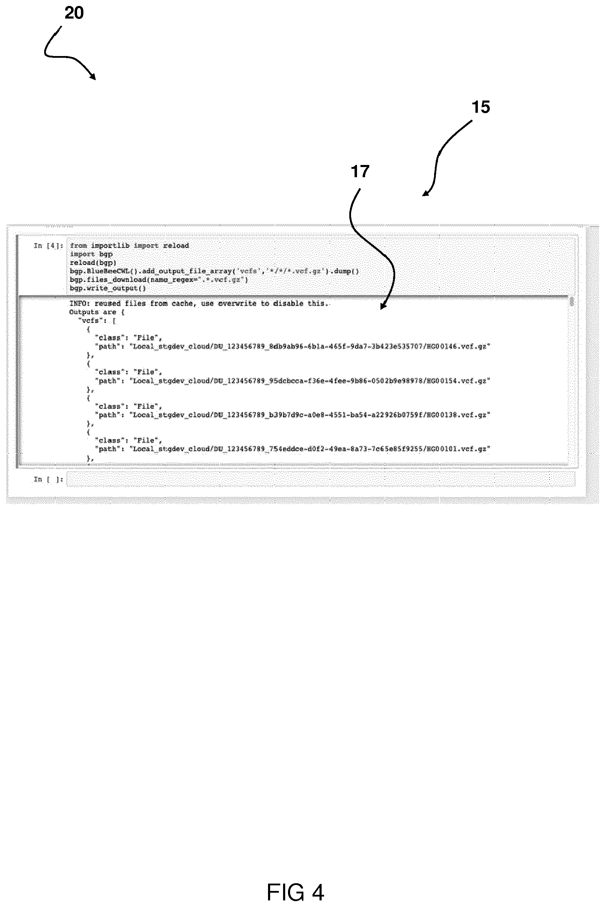

Fig. 4 shows a schematic diagram of an embodiment of a node configuration. The system may provide for a scientific notebook 15 for adjusting the node configuration. In this scientific notebook 15 parameters and operational tasks (cf. programming code 17) can be adjusted. The scientific notebook 15 may provide for a user interface 20. Different kinds of scientific notebook interfaces can be used, allowing changing of parameters and/or operational tasks relating to one or more nodes.

A node 3 may for instance have already defined operational tasks in the form of certain programming code. The system enables a user to edit the programming code (cf. operational tasks) of the node during execution of the graph, and even during execution of the particular node. The programming code may for instance be edited by pushing the edit button of the node 3 (see e.g. fig. 3). After the editing has finished, the node can be executed using the changed/updated programming code. In some examples, the processing of the graph 1 is temporarily paused during editing of the operational tasks. The execution of the node can then proceed after the editing has been finished. In some examples, the user can click a run button 9 for continuing the process.

In this example, the node configuration includes Python code for defining operational tasks. However, other programming languages may also be used for editing the operational tasks and/or parameters of the nodes 3. Various implementations are possible.

Different types of nodes 3 can be implemented. For example, a node 3 may relate to various data processing operations, such as for example performing principal component analysis (PCA), filtering data, ordering data, collecting data in certain formats, data matching, mapping, indexing of genetic reference sequences, etc. Other processing operations are also possible for example relating to pre-processing, post-processing, etc. In some examples, a node 3 may represent machine learning or artificial intelligence training sub process. In the training process, parameters can be adjusted dynamically, not requiring a complete rerun or re-initialization of the workflow or graph 1. At least a portion of the already processed result data being available can be used.

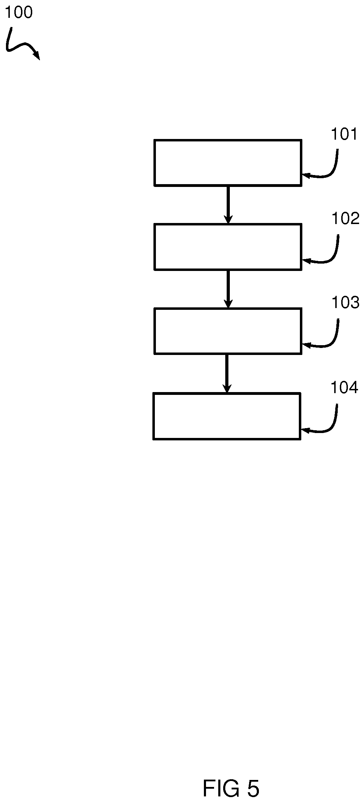

Fig. 5 shows a schematic diagram of a computer implemented method 100 for bioinformatics data analysis by processing computational data analysis workflows. In a first step 101, a computational workflow formed by a graph is received. The graph includes a graph structure with nodes and edges, wherein the edges in the graph indicate a flow of data between linked nodes. The nodes have a node configuration including at least a set of parameters and/or operational tasks, wherein the node configuration of the nodes is adjustable by a user. In a second step 102, input data is obtained and processing steps are performed on the input data by execution of nodes of the graph structure, wherein processed result data from the processing steps is stored. In a third step 103, a node configuration update of a selected node is performed based upon a received node configuration adjustment by the user while performing the processing steps during processing of the computational data analysis workflow. Additionally or alternatively, a graph structure update is performed based upon a received graph structure adjustment by the user, while performing the processing steps during processing of the computational data analysis workflow. In a fourth step 104, processing of the computational data analysis workflow is continued based on updated node configuration of the selected node and/or based on updated graph structure, wherein at least a portion of already processed result data being available is used during continued processing.

The graph can thus be tweaked in an efficient manner. Used algorithms of nodes can be efficiently modified while the graph is running and one or more nodes of the graph are being executed, while using already available result data.

Although various examples are provided relating to bioinformatics and health data analysis, it will be appreciated that the system and method may involve analysis of any kind of data.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the spirit and scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

## Claims

1. A bioinformatics data analysis system for processing computational data analysis workflows, the data analysis system comprising a computing unit including a processor coupled to a memory operable to cause the system to:
- receive a computational workflow formed by a graph, wherein the graph has a graph structure with nodes and edges, wherein the edges in the graph structure indicate a flow of data between linked nodes, and wherein the nodes have a node configuration including at least a set of parameters and/or operational tasks, wherein the graph structure and/or the node configuration of the nodes are adjustable by a user;
- obtain input data and perform processing steps on the input data by execution of nodes of the graph structure, wherein processed result data from the processing steps is stored;
- perform a node configuration update of a selected node based upon a received node configuration adjustment by the user, and/or perform a graph structure update based upon a received graph structure adjustment by the user, wherein the node configuration update and/or graph structure update are carried out while performing the processing steps during processing of the computational data analysis workflow; and
- continue processing of the computational data analysis workflow based on updated node configuration of the selected node and/or updated graph structure using at least a portion of already processed result data being available.

2. System according to claim 1, wherein the system is configured to enable a user to examine a status of each node while performing the processing steps during the processing of the computational data analysis workflow.

3. System according to claim 1 or 2, wherein the system is configured to enable a user to skip further execution of an active node by discontinuing execution of the active node and continuing execution of a subsequent node in the graph using the already processed result data being available.

4. System according to any one of the preceding claims, wherein upon updating the node configuration of a selected node, the system dynamically adjusts dedicated computational resources for performing the processing steps during the processing of the computational data analysis workflow.

5. Method according to any one of the preceding claims, wherein the system is further configured to determine whether the selected node in the graph has already been processed, wherein at least in case execution of the selected node in the graph is still ongoing or pending, processing of the computational data analysis workflow is continued based on the updated node configuration of the selected node.

6. System according to any one of the preceding claims, wherein in case the selected node has already been processed, the updated node configuration is used when the selected node is subsequently executed.

7. System according to any one of the preceding claims, wherein the system is configured to return processing of the computational data analysis workflow to execution of the selected node in case the selected node has already been processed.

8. System according to any one of the preceding claims, wherein the system is further operable by the user to change at least one of the nodes or edges of the graph while performing the processing steps during processing of the computational data analysis workflow, wherein processing of the computational data analysis workflow is continued based on the adjusted at least one of the nodes or edges of the graph while using the at least portion of already processed result data being available.

9. System according to any one of the preceding claims, wherein the bioinformatics data analysis system is configured to execute a sequence analysis pipeline.

10. System according to any one of the preceding claims, wherein the graph includes at least one node relating to machine learning.

11. System according to claim 10, wherein the at least one node relating to machine learning is at least one of: a training node configured to take an input and output a trained model, or a predictor node configured to take a trained model as input for obtaining a prediction as output.

12. System according to any one of the preceding claims, further including performing a compatibility check when operational tasks of the selected node are adjusted, wherein the compatibility check includes comparing at least data inputs provided through edges to the selected node in a reconfigured state to that of a previous state prior to the reconfiguration.

13. System according to any one of the preceding claims, wherein the graph is executed in an asynchronous process.

14. A computer implemented method for bioinformatics data analysis by processing computational data analysis workflows, the method comprising the steps of:
- receive a computational workflow formed by a graph, wherein the graph has a graph structure with nodes and edges, wherein the edges in the graph indicate a flow of data between linked nodes, and wherein the nodes have a node configuration including at least a set of parameters and/or operational tasks, wherein the graph structure and/or the node configuration of the nodes are adjustable by a user;
- obtain input data and perform processing steps on the input data by execution of nodes of the graph structure, wherein processed result data from the processing steps is stored;
- perform a node configuration update of a selected node based upon a received node configuration adjustment by the user, and/or perform a graph structure update based upon a received graph structure adjustment by the user, wherein the node configuration update and/or graph structure update are carried out while performing the processing steps during processing of the computational data analysis workflow; and
- continue processing of the computational data analysis workflow based on updated node configuration of the selected node and/or updated graph structure using at least a portion of already processed result data being available.

15. A computer program product configured for performing, when run on a controller, the steps of the method according to claim 14.
